# EUROPEAN PATENT APPLICATION

(11) **EP 3 476 300 A1**
(43) Date of publication of application: **01.05.2019**
(21) Application number: 17814519.9
(22) Date of filing: 11.05.2017
(51) Int. Cl.: A61B 17/00, A61B 90/00

(54) **MEMBRANE BREAKING DEVICE FOR ENDOVASCULAR SURGERY**

(30) Priority: 22.06.2016 CN 201610458021
(71) Applicant: Suzhou Innomed Medical Device Co., Ltd, Suzhou, Jiangsu 215123 (CN)
(72) Inventor: QU, Lefeng, Suzhou Jiangsu 215123 (CN); BAI, Jun, Suzhou Jiangsu 215123 (CN); HE, Ming, Suzhou Jiangsu 215123 (CN); GONG, Xiaoyan, Suzhou Jiangsu 215123 (CN); WEI, Jichang, Suzhou Jiangsu 215123 (CN)
(74) Representative: HGF Limited
(86) International application number: PCT/CN2017/083940
(87) International publication number: WO 2017/219781

(57) **Abstract**

A membrane breaking device for an endovascular surgery. The membrane breaking device comprises a membrane needle (1), a double-layer sheath (2), and a positioning support (3). The double-layer sheath (2) comprises an inner sheath (21) nested outside the membrane breaking needle (1) and an outer sheath (22) nested outside the inner sheath (21). The inner sheath (21), outer sheath (22) and the membrane breaking needle (1) can relatively move along the axial directions. The positioning support (3) is woven by at least one weaving filament. The far end of the positioning support (3) is connected to the inner sheath (21), the near end of the positioning support (3) is connected to the outer sheath (22), and the expansion and contraction of the positioning support (3) is controlled by controlling the relative movement between the inner sheath (21) and the outer sheath (22). The membrane breaking device can be used for covered stmt in-vivo perforation, and is used for a reconstructing a branch vessel of an aortic arch in the aortic dissection/endovascular aneurysm repair. The membrane breaking device can be also used for breaking the membrane type Budd-Chiari syndrome. The membrane breaking device has good flexibility and the blood vessel passing capability, can satisfy various requirements and can safely and accurately breaking a membrane.

## Description

### CROSS REFERENCE TO RELATED APPLICATIONS

This application claims the benefit of Chinese patent application No. CN 201610458021.7 filed Jun. 22, 2016, the entire disclosure of which is incorporated herein by reference.

### TECHNICAL FIELD

Described are medical devices, in particular, membrane puncturing devices for endovascular surgery.

### BACKGROUND

Aortic dissection occurs when a tear in the tunica intima of the aorta causes blood to flow between layers of the wall of the aorta, forcing the layers apart. Endovascular repair of aortic dissection in domestic and foreign vascular surgery centers has been carried out. A surgery needs to establish a channel approaching from the femoral artery, guide a guidewire to the breaking part of the dissection, implant a stent graft to the breaking part of the dissection, thereby covering the breaking part and expanding the true lumen to restore normal hemodynamics and achieve the purpose of repairing the dissection. However, the distance between an aortic rupture and a branch of the aortic arch (left clavicle artery, carotid artery, anonyma) is less than 1 cm; or in other cases, the aneurysm can overgrow over the aortic arch. The branch of the aortic arch will be covered during endovascular treatment, because this is needed to reconstruct the branch of the aortic arch.

Budd-Chiari syndrome refers to the obstruction of hepatic venous reflux resulting from stenosis or complete obstruction of the retrohepatic segment of the inferior vena cava and/or hepatic veins. The common types of Budd-Chiari syndrome include stenosis or obstruction of the inferior vena cava. Budd-Chiari syndrome can be further grouped into inferior vena cava simple diaphragm type, limited stenosis of the inferior vena cava, and limited obstruction of the inferior vena cava. Moreover, other types of Budd-Chiari syndrome include diffusion stenosis or obstruction of the inferior vena cava commonly caused by the formation of massive thrombosis and stenosis, or obstruction of hepatic veins. Interventional therapy with a small wound has been widely accepted as a solution for patients with incomplete or complete proximal membranous obstruction of the inferior vena cava, segmental stenosis of the inferior vena cava, no thrombus at locations distal of obstruction site, and unobstructed hepatic veins.

For this kind of interventional therapy involving passing through the diaphragm, the prior art interventional therapy includes puncturing a membrane of a stent, a site\s of stenosis, using a membrane puncturing needle directed by one or more guide wires, which thus allows the guide wires to pass through the membranes and direct further balloon expansion, and as a result the stent is implanted or the stenosis is eliminated. To note, it is important to ensure that the needle is directed accurately to the center of the membrane with appropriate strength when utilizing a membrane puncturing needle. If the needle is incorrectly directed to the periphery of the membrane instead of the center of the membrane, the needle may break the vessel. Moreover, as there is normally a long distance from the puncturing point to the membrane site. Due to insufficient support of the current medical devices, superior difficulty in adjusting the strength, lack of control of the accurate location, and length of puncturing needle, prior art treatment methods face challenges when puncturing the membrane via membrane puncturing needles. With regard to these challenges, utilization of prior art membrane puncturing needles may have disadvantages such as risks of breaking the membrane, long operation periods, relatively low success ratio, and high risks of accidental injury. During operation, inaccurate puncture and over-puncture may break veins, or even accidentally puncture into pleura or pericardium leading to unnecessary injury.

### SUMMARY

Considering the shortcomings membrane puncturing needles in the prior art, the present improved devices provide a membrane puncturing device with much better flexibility and vessel passability, which can achieve better results and allow for more safe and accurate membrane puncture.

The described membrane puncturing device includes a membrane puncturing needle, a double-layer sheath, and a positioning stent. The double-layer sheath includes an inner sheath sleeved outside the membrane puncturing needle and an outer sheath sleeved outside the inner sheath, wherein the inner sheath, the outer sheath, and the membrane puncturing needle can move along an axial direction relative to each other. The positioning stent is woven by at least one wire. The distal end of the positioning stent is connected to the inner sheath and the proximal end of the positioning stent is connected to the outer sheath. Expansion and contraction of the positioning stent can be controlled by relative movement between the inner sheath and the outer sheath.

In one embodiment, a radiopaque marker is included in the device. The radiopaque marker includes at least one of a radiopaque wire, a radiopaque ring, a radiopaque point. In some embodiments, the radiopaque wire is contained in at least one wire, the radiopaque ring is disposed on the positioning stent, the radiopaque point coated on the wire, and combinations thereof.

In another embodiment, the distal end of the positioning stent is fixed with the inner sheath by hot-melt adhesion and/or the proximal end of the positioning stent is fixed with the outer sheath by hot-melt adhesion.

In still another embodiment, the positioning stent is woven by crossing-overlapping at least one wire. The wires peripherally adjacent to each other form diamond grid units. The obtuse angles of the diamond grid units range from 91 to 179 degrees, from 100 to 160 degrees, from 110-150 degrees, from 115-140 degrees, or from 120-135 degrees. The shape of the cross section of the wires is selected from the group consisting of round, ellipse, trapezoid, diamond, or rectangle. The wire is made from at least one of nickel-titanium alloy, 304 stainless steel, 316L stainless steel, L605 cobalt-chromium alloy, and MP35N alloy. The wire diameter of the at least one wire ranges from 0.005 inches to 0.02 inches. The lay of braiding of the positioning stent ranges from 0.6 mm to 1.5 mm. The maximum outer diameter of the positioning stent, when it is expanded, ranges from 5 mm to 40 mm. The positioning stent can be a spherical stent, a fusiform stent, a cylindrical stent, or a conical stent. Alternatively, the positioning stent is formed by forward braiding and backward braiding the at least one wire.

In another embodiment, the membrane puncturing needle has a puncturing tip for puncturing a membrane and the puncturing tip has a radiopaque marker. Moreover, the end opposite of the puncturing tip of the membrane puncturing needle has a scale marker. In some embodiments, the membrane puncturing device also has a limiting apparatus that is engaged with the end opposite of the puncturing tip of the membrane puncturing needle for limiting movement of the membrane puncturing needle. The membrane puncturing needle is hollow, and the hollow cavity can be used for allowing guidewires to pass there through. The membrane puncturing needle has an outer diameter ranging from 0.3 mm to 1.5 mm. Alternatively, the membrane puncturing needle is a flexible optical fiber.

In other embodiments, the membrane puncturing device also includes a locking apparatus that is connected to the proximal end of the inner sheath. The locking apparatus is for locking the distance between the distal end of the inner sheath and the distal end of the outer sheath.

The present devices have the following advantageous effects over the prior art. The presently described positioning stent is woven by at least one wire, such that the positioning stent is not breakable, has enhanced tensile strength, has excellent radial positioning capability and flexibility, and thus it will adapt well to vessels.

The woven positioning stent has a hollowed-out structure (the wires adjacent to each other peripherally form diamond grid units), and therefore, the stent does not influence blood flow when the membrane is punctured. And the helical and grid structures of the woven stent itself have an excellent anchoring effect to prevent shifting of the positioning stent when puncturing the membrane.

The positioning stent is gradually released so as to reduce damage on the inner wall of the vessel.

Meanwhile, the radiopaque marker disposed on the positioning stent (e.g., radiopaque wire, radiopaque ring, radiopaque point, and the like) may make the positioning stent clearly visible in the body.

In addition, the positioning stent may be connected to the double-layer sheath by hot-melt adhesion. That is to say, the hot-melt adhesion is performed on the inner and the outer sheaths themselves. In comparison with conventional glue adhesion, such hot-melt adhesion not only has high connection strength, great tensile strength, and excellent passability, but also has no potential risk from the addition of other components.

The radiopaque marker is disposed on the distal end of the membrane puncturing needle (puncturing tip) such that the distal end of the membrane puncturing needle can be clearly visible, thereby accurately puncturing the membrane. The scale marker is disposed on the proximal end of the membrane puncturing needle (the end opposite to the puncturing tip) to indicate membrane crossing length, and the limiting apparatus is arranged to control membrane crossing length, such that damage caused by over-puncture can be avoided, thereby accurately and safely puncturing the membrane. Further, the hollow cavity of the membrane puncturing needle allows the guidewire to pass there through, such that the guidewire can be further deployed after puncturing the membrane, so as to provide convenience for further operation.

The described membrane puncturing devices for endovascular surgery, such as diaphragm-type Budd-Chiari syndrome and opening a hole on a membrane covered stent in vivo, have excellent flexibility and vessel passability, which can meet various requirements and safely and accurately puncture the membrane. Meanwhile, since the positioning stent and the distal end of the membrane puncturing needle are radiopaque and the limiting apparatus and the scale marker are connected to the proximal end of the membrane puncturing needle, the membrane can be punctured accurately and safely.

### BRIEF DESCRIPTION OF DRAWINGS

FIG. 1 is a schematic drawing of the membrane puncturing device according to one embodiment of the present description.
FIGS. 2a and 2b are schematic drawings during use of the membrane puncturing device as shown in FIG. 1.
FIG. 3 is an enlarged schematic drawing of the membrane puncturing device in FIG. 1, when the membrane puncturing device punctures the membrane.

### DETAILED DESCRIPTION

Several aspects of the invention are described below in details with reference to appended drawing and specific embodiments for illustration. Members in the drawings are used to illustrate the principles of the present invention.

The structures and/or the materials in the examples of the present description, which are well known in the art, are not shown or described in detail. The features, structures, and/or characteristics may combine together in one or more embodiments. In addition, those skilled in the art would understand that the embodiments are set forth to provide a full understanding of the invention, rather than limit the protection scope thereof. Those skilled in the art would also understand that the components in the embodiments, as mentioned and as shown in the drawings, can be arranged or designed in different ways or at different scales.

### First Embodiment

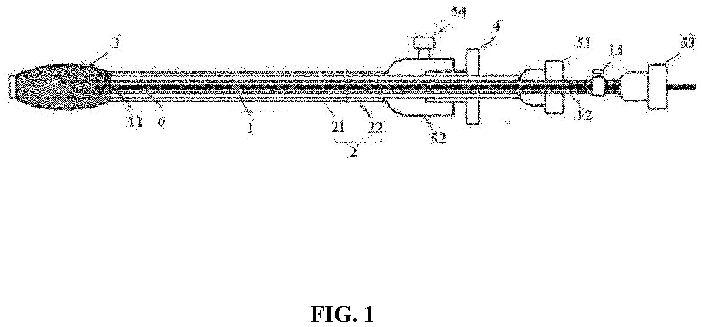

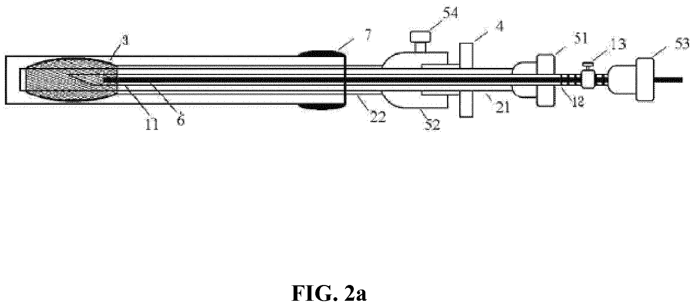

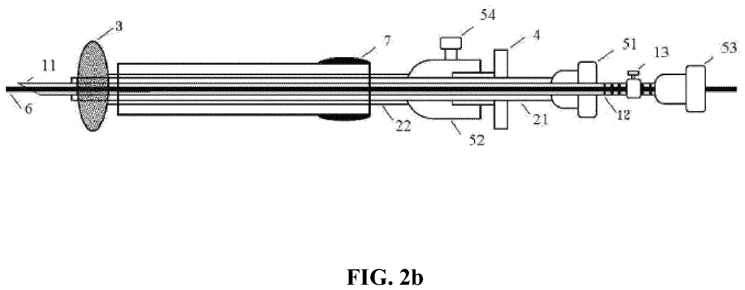

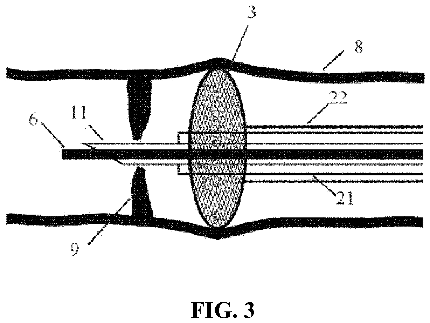

FIG. 1 is a schematic drawing of the membrane puncturing device according to one embodiment. FIGS. 2a and 2b are schematic drawings during use of the membrane puncturing device as shown in FIG. 1. FIG. 3 is an enlarged schematic drawing of the membrane puncturing device of FIG. 1 when the membrane puncturing device punctures a membrane.

As shown in FIG. 1, the membrane puncturing device includes a membrane puncturing needle 1, a double-layer sheath 2, and a positioning stent 3, in which, the double-layer sheath includes an inner sheath 21 sleeved outside of the membrane puncturing needle and an outer sheath 22 sleeved outside of the inner sheath. The inner sheath 21 and the outer sheath 22, as well as the membrane puncturing needle 1, can move along an axial direction relatively to each other. Specifically, the inner sheath 21 and the outer sheath 22 can move along the axial direction relatively to each other, and the membrane puncturing needle 1 and the inner sheath 21 can move along the axial direction relatively to each other. The positioning stent 3 is woven by at least one wire. The distal end of the positioning stent 3 is connected with the inner sheath 21, and the proximal end of the positioning stent 3 is connected with the outer sheath 22. The expansion and contraction of the positioning stent can be controlled by relative movement between the inner sheath and the outer sheath. In one embodiment, the outer sheath 22 has a length shorter than the inner sheath 21, the distal end of the positioning stent 3 is connected with the distal end part of the inner sheath 21, and the proximal end of the positioning stent 3 is connected with the distal end of the outer sheath 22. When the distal ends of the double-layer sheath are pulled out a certain distance with each other, the positioning stent is in a contracted state. When the distal ends of the double-layer sheath are drawn close to each other by an external force(s), the positioning stent is in an expanded state. The membrane puncturing needle 1 has a length longer than the double-layer sheath, and the membrane puncturing needle 1 is moved when the positioning stent is in expansion state, such that the puncturing tip of the membrane puncturing needle extends out of the distal end of the inner sheath 21 and thus punctures a corresponding membrane.

In some embodiments, the positioning stent 3 includes a radiopaque marker. In one embodiment, the radiopaque marker may include a radiopaque wire contained in at least one wire, a radiopaque ring disposed on the positioning stent (e.g., the radiopaque ring may be disposed on the distal and proximal ends of the stent, as C-type ring or round ring), a radiopaque point applied on the wire, and a combination thereof. The radiopaque marker may be other markers which are able to make the positioning stent radiopaque. The radiopaque feature of the positioning stent may be used for indicating the location of the positioning stent in the body so as to ensure that the membrane puncturing needle accurately punctures the membrane.

In this embodiment, the positioning stent 3 is woven through crossing-overlapping at least one wire. The wires peripherally adjacent to each other form a diamond grid unit. In some embodiments, the crossing-overlapping refers to the circumstance that wire a is above wire b when wire a contacts wire b at the first time, and when wire a contacts wire b at the second time, wire a pass below wire b and thus they cross with each other. The obtuse angle of the diamond grid unit ranges from 91 to 179 degrees, from 100 to 160 degrees, from 110 to 150 degrees, from 115 to 140 degrees, or from 120 to 135 degrees. In one embodiment, the obtuse angle of the diamond grid unit ranges from 120-135 degrees. The lay of braiding of the positioning stent (i.e., the straight-line distance between two cross points along the axial direction) is between 0.6 mm and 1.5 mm, and in some embodiments 1.0 mm (in the contracted state as shown in FIG. 1). The wire has a wire diameter ranging from 0.005 inches to 0.02 inches. The shape of the cross section of the wires can be round, an ellipse, trapezoidal, diamond, or a rectangle. The wire is made from at least one of nickel-titanium alloy, 304 stainless steel, 316L stainless steel, L605 cobalt-chromium alloy, and MP35N alloy. In one embodiment, nickel-titanium alloy can make the woven positioning stent have increased radial strength for positioning and great flexibility. The positioning stent can be a spherical, fusiform, cylindrical, or conical stent. The maximum outer diameter of the positioning stent when being expanded (i.e., the expansion state of the positioning stent 3 as shown in FIGS. 2b and 3) is between 5 mm and 40 mm.

In this embodiment, the positioning stent is formed without braiding at forward and backward directions (i.e., the wires firstly wind via front to back and then wind in reverse). Two ends (free ends) of the wires are located on two ends of the positioning stent, which can be fixed with the inner sheath 21 and the outer sheath 22. In other embodiments, the positioning stent 3 can be formed by forward and backward braiding the at least one wire, at this time, two ends (i.e., radial ends) of the positioning stent are not open, both of which are fixed with the inner sheath 21 and the outer sheath 22, respectively.

In this embodiment, the distal end of the positioning stent is fixed with the inner sheath 21 by hot-melt adhesion and the proximal end of the positioning stent is fixed with the outer sheath 22 by hot-melt adhesion. In some embodiments, the hot-melt adhesion means that, for example, the distal end of the inner sheath is heated and partially molten and then the distal end of the positioning stent is extended into the molten part of the distal end of the inner sheath, followed by being pressed and cooled, such that the distal end of the positioning stent can be fixed with the distal end of the inner sheath. In comparison with common connections using glue, the hot-melt adhesion does not need new adhesive materials, because it uses the sheath itself for adhesion instead. Therefore, the hot-melt adhesion has a high connection strength and no potential risk, which is rendered due to the introduction of other components for the clinic environment. Nevertheless, the positioning stent can be fixed with the inner sheath 21 and the outer sheath 22 respectively using other adhesion methods. For example, medically acceptable adhesives can be used to fix the positioning stent with the inner sheath and the outer sheath.

As shown in FIG. 1, the membrane puncturing needle 1 may have a puncturing tip 11 (distal end) for puncturing a membrane. Such a puncturing tip 11 may have a radiopaque marker. In some embodiments, the radiopaque marker may be a point or a ring formed by applying the radiopaque material to the puncturing tip, or other markers that can make the puncturing tip visible. The other end (proximal end) of the membrane puncturing needle, which is opposite of the puncturing tip 11, has a scale marker 12. Moreover, the membrane puncturing device has a limiting apparatus 13 that is engaged with the other end (proximal end) of the membrane puncturing needle, for limiting the movement of the membrane puncturing needle. The limiting apparatus 13 may include a nut groove and a fixing nut. The fixing nut can be tightened in the nut groove, such that the fixing nut closely contacts the membrane puncturing needle to increase the friction force between them, thereby fixing one to the other. The length through which the membrane puncturing needle crosses the membrane can be indicated by the scale marker (length marker) on the proximal end. After the length is determined, the fixing nut is tightened to limit the movement of the membrane puncturing needle, such that the length can be fixed without changing.

The outer diameter of the membrane puncturing needle ranges from 0.3 mm to 1.5 mm. In one embodiment, the membrane puncturing needle is hollow, and the hollow cavity allows guidewires 6 to pass therethrough. In another embodiment, the membrane puncturing needle is a flexible optical fiber and it is used to puncture the membrane by laser.

The membrane puncturing device also includes a locking apparatus 4 connected with the proximal end of the inner sheath, for locking the distance between the distal end of the inner sheath and the distal end of the outer sheath. In some embodiments, he locking apparatus 4 is a fastening screw. The inner sheath can be locked and remain fixed by tightening such a fastening screw, such that the distance between the inner sheath 21 and the distal end of the outer sheath 22 can be fixed.

The membrane puncturing device also includes an inner sheath joint 51, an outer sheath joint 52, and a membrane puncturing needle joint 53. The inner sheath joint is connected with the proximal end of the inner sheath, the outer sheath joint is connected with the proximal end of the outer sheath, and the membrane puncturing needle joint is connected with the proximal end of the membrane puncturing needle. These joints are convenient for doctors to move the inner sheath, the outer sheath, and the membrane-puncturing needle when performing operations. In some embodiments, a washing hole 54 is disposed on the outer sheath joint 52, for washing and imaging in the double-layer sheath.

In addition, as shown in FIGS. 2a and 2b, when using the membrane puncturing device, dilator sheath 7 (i.e., vascular dilator) would be firstly introduced, through which the inner sheath and the outer sheath are delivered to the corresponding position.

Referring to FIG. 3, the membrane puncturing device is delivered to the target position in the vessel 8, and then the inner sheath 21 and the outer sheath 22 are operated to expand and fix the positioning stent 3. The membrane puncturing needle 1 is operated to pass through the target membrane 9 (it can be intravascular diaphragm or stenosis caused by Budd-Chiari syndrome, or can be the membrane of a membrane-covered stent), such that the membrane is punctured.

The membrane puncturing device can be disposable or can be packaged in bags, boxes, or other suitable containers after sterilizing by any suitable technique. For example, the disposable membrane puncturing device can be packaged with a protective coil for sale. After taking out the membrane puncturing device, it can be cleaned by normal saline.

### Second Embodiment

Embodiments also provide a method for operating the above membrane puncturing device, including the following steps:
a. Allowing the hollow puncturing needle to puncture into the body and then arrive at the target position via vessels. In this step, firstly, the skin at the puncturing site should be routinely sterilized. Secondly, topical anaesthesia is performed. Next, the skin at the puncturing site is cut by a scalpel to form an incision between about 2 mm and 3 mm, followed by allowing the puncturing needle to puncture into the incision of the skin. The proximal end of the target artery is pressed by the left hand of the operator and the needle is entered at a required oblique angle. If pulsatile blood flow can be seen, it means the tip of the needle is located within the artery and then the core of the needle is withdrawn.
b. Introducing the puncturing guidewire into the vessel through the tail of the puncturing needle. During insertion of the puncturing guidewire, the puncturing needle is gradually withdrawn. After ensuring that the puncturing guidewire enters into the artery, the puncturing needle is withdrawn and the puncturing site is pressed by the hand to prevent bleeding.
c. Directing the dilator sheath 7 (vascular dilator) to the target position in the vessel by the introduction of the puncturing guidewire, followed by withdrawing the puncturing guidewire. During the operation, it should be noted that the puncturing guidewire must be exposed out of the tail of the dilator sheath 7 so as to avoid falling of the puncturing guidewire into the body. After the dilator sheath 7 is delivered into the artery, the puncturing guidewire is withdrawn.
d. Delivering the guidewire 6 to the target position through dilator sheath 7 and then fixing the guidewire 6.
e. Delivering the inner and the outer sheaths of the membrane puncturing device to the target position along the guidewire 6 through dilator sheath 7. During the delivery of the inner and the outer sheaths, the operator injects heparinized normal saline discontinuously.
f. Pushing the membrane puncturing device to the membrane puncturing site, releasing positioning stent 3, and locking the radial length and maximum expansion outer diameter of the stent. During operation, it should be noted that the positioning stent can't be expanded and fixed unless the location is determined. Referring to FIGS. 2a and 2b, it can be seen that the positioning stent is released and expanded, and then fixed.
g. Withdrawing the guidewire 6 into membrane puncturing needle 1 and gradually pushing the membrane puncturing needle to the target position until the membrane is punctured. This process needs to use the scale marker 12 and limiting apparatus 13 at the proximal end of the membrane puncturing needle to ensure that the membrane puncturing needle is delivered to an accurate position and fixed. If the membrane has not been punctured successfully, the limiting apparatus at the proximal end needs to be re-adjusted to puncture the membrane again. In this step, guidewire 6 is withdrawn into the membrane puncturing needle 1 so as to avoid damaging the guidewire or having an influence on membrane puncture when pushing the membrane puncturing needle. The limiting apparatus at the proximal end of the membrane puncturing needle is adjusted to an appropriate position and fixed firmly to avoid the membrane puncturing needle over crossing the membrane thereby damaging vessel on the opposite side and sounding tissues.
h. Delivering the guidewire 6 after puncturing the membrane (the guidewire 6 is extended into the broken hole of the membrane) and withdrawing the membrane puncturing needle after the guidewire passes through the broken hole.
i. Contracting the positioning stent by adjusting the distance between the distal end of the inner sheath and the distal end of the outer sheath, and then gradually withdrawing the positioning stent along the inner and the outer sheaths and pulling out of the membrane puncturing device along the dilator sheath 7.
j. Selecting a suitable balloon and allowing it to arrive at the broken hole for expansion, after the membrane is punctured. Then, the subsequent work is carried out.

During an operation, the puncturing needle, scalpel and sheath and the like need to be sterilized prior to using membrane puncturing device. And since the guidewire and the protective coil for the guidewire are sold together, sufficient heparinized normal saline should be injected into the coil using the syringe prior to taking the guidewire out, so as to readily hydrate the guidewire's coating layer.

The terms as used herein should be considered as illustrative, but not restrictive. Those skilled in the art would understand that many modifications may be made without departing from the spirit thereof. Thus, the scope of the present invention should be defined by the accompanying claims. Unless defined otherwise, all terms used herein should be understood as having their broadest meaning.

## Claims

1. A membrane puncturing device, comprising:
a membrane puncturing needle ,
a double-layer sheath, comprising an inner sheath sleeved outside of the membrane puncturing needle and an outer sheath sleeved outside of the inner sheath, the inner sheath, the outer sheath, and the membrane puncturing needle are configured to move along an axial direction relatively to each other; and
a positioning stent knitted by at least one wire comprising a distal end and a proximal end, the distal end of the positioning stent is connected to the inner sheath, the proximal end of the positioning stent is connected to the outer sheath, and expansion and contraction of the positioning stent is controlled by relative movement between the inner sheath and the outer sheath.

2. The membrane puncturing device of claim 1, wherein the positioning stent comprises a radiopaque marker.

3. The membrane puncturing device of claim 2, wherein the radiopaque marker comprises a radiopaque wire contained in the at least one wire, a radiopaque ring disposed on the positioning stent, a radiopaque point coated on the wire, or a combination thereof.

4. The membrane puncturing device of claim 1, wherein the distal end of the positioning stent is fixed with the inner sheath by hot-melt adhesion, and the proximal end of the positioning stent is fixed with the outer sheath by hot-melt adhesion.

5. The membrane puncturing device of claim 1, wherein the at least one wire includes multiple wires the positioning stent is woven by crossing-overlapping the multiple wires, and the multiple wires peripherally adjacent to each other form diamond grid units.

6. The membrane puncturing device of claim 5, wherein diamond grid units comprise an obtuse angle, wherein the obtuse angle ranges from one of followings: 91 to 179 degrees, 100 to 160 degrees, 110 to 150 degrees, 115 to 140 degrees, 120 to 135 degrees.

7. The membrane puncturing device of claim 5, wherein the positioning stent comprises a lay of braiding between 0.6 mm and 1.5 mm.

8. The membrane puncturing device of claim 1 or 5, wherein the positioning stent is formed by forwards and backward braiding the at least one wire.

9. The membrane puncturing device of claim 1, wherein the at least one wire is made from at least one of nickel-titanium alloy, 304 stainless steel, 316L stainless steel, L605 cobalt-chromium alloy, and MP35N alloy.

10. The membrane puncturing device of claim 1, wherein the at least one wire has a wire diameter ranging from 0.005 inches to 0.02 inches.

11. The membrane puncturing device of claim 1, wherein the positioning stent has a maximum outer diameter of between 5 mm and 40 mm when expanded.

12. The membrane puncturing device of claim 1, wherein the positioning stent is spherical, fusiform, cylindrical, or conical.

13. The membrane puncturing device of claim 1, wherein the membrane puncturing needle has a puncturing tip for puncturing the membrane and the puncturing tip has a radiopaque marker.

14. The membrane puncturing device of claim 13, wherein an end opposite to the puncturing tip of the membrane puncturing needle has a scale marker.

15. The membrane puncturing device of claim 14, wherein the membrane puncturing device comprises a limiting apparatus for limiting the movement of the membrane puncturing needle that is engaged with the end opposite to the puncturing tip of the membrane puncturing needle.

16. The membrane puncturing device of any of claim 1, claim 13 and claim 14, wherein the membrane puncturing needle comprises a hollow cavity and the hollow cavity is configured to allow a guidewire to pass therethrough.

17. The membrane puncturing device of claim 16, wherein the membrane puncturing needle has an outer diameter between 0.3 mm and 1.5 mm.

18. The membrane puncturing device of claim 1, wherein the membrane puncturing device further comprises a locking apparatus that is connected to the inner sheath at a proximal end, the locking apparatus is configured to lock the distance between a distal end of the inner sheath and a distal end of the outer sheath.

19. The membrane puncturing device of claim 1, wherein the membrane puncturing needle is a flexible optical fiber.
